# EUROPEAN PATENT SPECIFICATION

(11) **EP 0 508 209 B1**
(45) Date of publication and mention of the grant of the patent: **29.12.1997**
(21) Application number: 92105072.0
(22) Date of filing: 24.03.1992
(51) Int. Cl.: A61N 5/06

(54) **Apparatus for inducing tanning or DNA repair by pulsed radiation**
Vorrichtung zum Hervorrufen von Bräunung oder DNA-Reparatur durch gepulste Strahlung
Dispositif pour induire le bronzage ou la réparation du DNA par radiation pulsatoire

(30) Priority: 27.03.1991 US 675689; 10.01.1992 US 819116
(43) Date of publication of application: 14.10.1992
(73) Proprietor: Changaris, David G., Louisville, Kentucky 40205 (US)
(72) Inventor: Changaris, David G., Louisville, Kentucky 40205 (US)
(74) Representative: Grünecker, Kinkeldey, Stockmair & Schwanhäusser Anwaltssozietät

(56) References cited:
- EP-A- 0 387 423
- WO-A-91/13652
- DE-A- 3 734 852
- US-A- 4 112 335
- US-A- 4 862 886

## Description

This invention relates to a new technique to induce "tanning" in humans, by utilizing very short, discrete pulses of electromagnetic radiation of selected wave length, for example about 250-400 nanometers ("nm"), preferably about 280-300 nm with an optimum of about 290 nm. The invention in a second embodiment is also capable of enhancing the repair of ultraviolet energy-damaged deoxyribonucleic acid ("DNA") using electromagnetic radiation of a second selected wave length from about 320-600 nm, preferably above about 375 nm with an optimum of about 460-500 nm and a target of about 480 nm. Both embodiments may be combined into a single unit to selectively promote tanning or DNA repair, at the option of the user.

The prior art discloses several apparati to induce artificial tanning of human skin. See, US-A-4,862,886; 4,794,925; 4,711,448; 4,611,327 and 4,469,102. The prior art devices generally utilize a source of continuous fluorescent ultraviolet ("UV") radiant energy, usually within the Ultraviolet A ("UVA") spectrum (wave length 320 to 390 nm), with very limited amounts of Ultraviolet B ("UVB") spectrum (wave length, 286 to 320 nm); the Ultraviolet C spectrum ("UVC", wave length 40-286 nm) is seldom utilized in tanning machines.

WO-A-91/13652 published September 19, 1991 relates to an apparatus for treating abnormal pigmentation of the skin comprising a first pulsed laser having a wave length between 345 and 600nm for the treatment of epidermal pigmentation and a second pulse laser with a wave length between 600 and 1000nm for the treatment of dermal pigmented lesions.

US-A-4,112,335 relates to a pulse UV lamp for resin polymerization.

EP-A-0 387 423 discloses an apparatus comprising a light source, such as a conventional bulb, with a shutter for periodically blocking the light path of said light source.

Tanning occurs largely through a process known as melanogenesis, a process which turns skin darker by stimulating the melanin-containing cells known as melanocytes to generate small packets of pigment. When an individual increases the pigment in his or her skin the emotional result is generally a sense of well-being and perceived improved appearance. UVB light is a known initiator for melanogenesis with a peak "tanning efficiency" at a wave length of about 290 nm. However, UVB has heretofore been saddled with safety concerns. This is because, even though it is known that peak tanning efficiency occurs at about 290 nm, it is also true that exposure to UVB energy has a significant risk of the toxic result of erythema, or "sun burn". Because of this risk, United States Federal Regulations (e.g., 21 CFR §1040.20(ii)(c)(1)), place strict limitations on the amount of UVB radiation a recipient may be exposed to (usually, the total energy source must emit less than 0.3% UVB). Consequently, prior art tanning devices are ordinarily required to utilize UVA energy sources (e.g., a continuously emitting fluorescent light tube). As a result, currently available tanning booths and the like are required to use the least efficient source of radiant energy to promote tanning.

Light above but near 320 nm is within the UVA spectrum. UVA will induce the so called "immediate tanning" related to the oxidation of pre-existing melanin, the dark pigment within skin. It is thought that this stimulus of tanning is most likely dependent upon the minimal but still present UVB within commercially available tanning light sources. These currently available suntanning light sources use continuous light sources that produce a series of potentially toxic results, for example the rapid destruction of genetic (thymine dimer formation) and protein structure through the build up of cellular toxins, presumably superoxides and resulting products. Such toxic effects are often divided into both short and long term events. The short term events include sun burn, corneal clouding, and retinal damage. The long term effects include premature aging of the skin and accelerated cancer incidence (e.g., melanoma).

Accordingly, it is the object of the invention to provide an apparatus to promote suntanning in humans resulting from exposure to very short, discrete pulses of energy of selected wave length to promote suntanning, without consequent risk of sunburn or other toxic results.

The invention promotes suntanning in a very short elapsed time.

According to a preferred embodiment the invention provides an apparatus equipped with an energy source to provide very short discrete pulses of energy of multiple selected wave lengths to permit both suntanning and tissue repair, either simultaneously or separately.

According to a preferred embodiment of the present invention, there is provided an apparatus for tanning skin or repairing ultraviolet induced skin damage, said apparatus comprising: means defining a location at which the skin is to be positioned for tanning or repairing; and, at least one electromagnetic pulse generating means for directing discrete pulses of electromagnetic radiation towards said location defining means, said electromagnetic pulse generating means including continuous electromagnetic generating means for continuously emitting electromagnetic radiation in a path towards said location defining means and path blocking means for periodically blocking the path of the continuously emitted electromagnetic radiation to obtain the discrete pulses of electromagnetic radiation.

Some possible path blocking means comprise a plurality of parallel rotating slats located between said location defining means and said continuous electromagnetic generating means, each of the plurality of parallel rotating slats having an aperture therethrough to allow the passage of the continuously emitted electromagnetic radiation at a predetermined rotational position; one or more rotating cylinders located between said location defining means and said continuous electromagnetic generating means, each cylinder having an aperture therethrough to allow the passage of the continuously emitted electromagnetic radiation at a predetermined rotational position; one or more rotating hollow cylinders located around said continuous electromagnetic generating means, each cylinder having at least one aperture therethrough to allow the passage of the continuously emitted electromagnetic radiation at a predetermined rotational position toward said location defining means; and, a plurality of parallel rotatable members and means for rotating said plurality of parallel rotatable members, each of the plurality of rotatable members having at least one reflective surface for, when said rotatable member is rotated, periodically reflecting the continuously emitted electromagnetic radiation towards said location defining means.

According to another preferred embodiment of the present invention, there is provided an apparatus for tanning skin or repairing ultraviolet induced skin damage, said apparatus comprising: means defining a location at which the skin is to be positioned for tanning or repairing; and, at least one electromagnetic pulse generating means for directing discrete pulses of electromagnetic radiation towards said location defining means, said electromagnetic pulse generating means including at least one strobe light to intermittently produce electromagnetic radiation. Also, the electromagnetic pulse generating means can further comprise a filter means located between said at least one strobe light and said location defining means, for passing therethrough intermittent electromagnetic radiation of one or more selected wave length bands.

According to a further preferred embodiment of the present invention, there is provided in said electromagnetic pulse generating means of any of the above described apparatuses a first means for emitting electromagnetic radiation of said first selected wave length to induce tanning, and a second means for emitting electromagnetic radiation of said second selected wave length to effect repairing ultraviolet radiation-induced DNA damage within skin cells.

According to another feature of the invention, in the apparatuses described above, there are provided at least two electromagnetic pulse generating means and the discrete pulses directed towards said location defining means are synchronized.

Preferably, in all of the apparatuses described above, each discrete pulse of radiation has a pulse duration of about 1 picosecond to about 20 milliseconds and wherein the discrete pulses of radiation for tanning have a wave length of about 250 to 400 nanometers and the discrete pulses of radiation for repairing ultraviolet radiation-induced DNA damage within skin cells have a wave length of about 320 to 600 nanometers. Also, in all of the apparatuses described above, the electromagnetic pulse generating means can be selected which produce electromagnetic radiation of desired wave length(s), or which produce radiation of many wave lengths which is then filtered to pass only the desired wave length(s).

I have discovered that the tanning can occur with pulsed radiation, and that it is not necessary to have a continuous source of radiation to induce tanning. Surprisingly, by subjecting the skin to very short, discrete pulses of energy, it is possible to effect an inducement of the melanocytes to produce pigmentation changes equivalent to those produced by continuous irradiation sources, but without manifesting tissue injury (e.g., sunburn) that is so often associated with those prior art devices. Thus, the present invention emits short discrete pulses of electromagnetic energy which will permit tanning, but with significantly reduced injury to structural elements such as collagen and cells.

The exposure to pulsed radiation will induce tanning and/or repair of damaged DNA depending upon the wave lengths defined. Pulses on the order of picoseconds to milliseconds produce an irradiation cycle which will prevent buildup toxic products known to accumulate during continuous exposure. An important feature of the present invention is that pulsed energy also allows sufficient time between energy impingements for the resident enzymes within the irradiated area to eliminate light-induced toxic products, for example, superoxides, thymine dimers, oxidized light-sensitive proteins (i.e., the characteristic by-products of skin burning). Thus, the total accumulated exposure to the energy source and the absolute time required to achieve the desired tanning level is markedly reduced.

Pulsed energy irradiation of the appropriate wave length can also repair DNA dimers which have been damaged by excessive exposure to UV energy. Exposure to continuous light damages DNA by altering the thymine structure to produce thymine dimers; the rate of thymine dimer formation is thought to be critical to the integrity of skin. It is also known that continuous ultraviolet exposure causes thymine bases to become thymine dimers. Many have speculated that this in turn contributes to premature aging and increasing incidence of skin cancer.

The body's response to correct the formation of thymine dimers includes a process known as photoreactivation to produce an enzyme known as photolyase, and another process known as dark repair. The majority of thymine dimer repair is believed to occur through photolyase. The energy absorption characteristics of UV irradiation to produce or inhibit UV-induced skin damage are generally known. Photolyase production is driven most efficiently by blue to green light (wave length, 375-500 nm); dark repair occurs without light. In conducting experiments to test my invention as described herein, I have come to believe and understand that photoreactivation or photolyase can be induced or initiated with discrete pulses of ultraviolet energy in the high end of the UVA range (wave length, above 375 nm), even by a single discrete pulse. Ordinarily, continuous exposure to UV light leads to the accumulation of toxic by-products such as superoxides, which can and do degrade the photoreactive systems thought to be present in human photolyase. However, it appears that serial exposure to discrete pulses of UV energy in this range will not produce the usual toxic byproducts associated with continuous UV exposure provided by present tanning booths, but will in fact enhance the repair of thymine dimers. This is thought to be because, with a discrete pulse, there is a "dark" portion between the active portion of the energized pulse. Because there is a dark non-energized portion of each discrete pulse, the cumulative energy within each pulse is sufficient to induce photolyase, but not sufficient to induce or create the toxic by-products.

A device is provided whereby the cumulative energy input is limited to an amount which will be sufficient to stimulate the melanocytes within the skin to induce melanogenesis, but which will be insufficient to produce the toxic products known to be associated with excess exposure of the skin to UV irradiation.

Because the accumulation of toxic products usually associated with exposure to continuous energy sources is so vastly reduced when utilizing discrete pulses, with the present invention it will be possible to utilize the more efficient UVB energy source within its most efficient tanning range (for example, about 290 nm), without the heretofore feared toxic side effects. As stated above, I also contemplate that when my invention is utilized in certain selected energy wave lengths (for example, 375-500 nm), the discrete pulses are capable of repairing UV-induced skin damage. Different spectral characteristics of the energy/light source, or filtering devices, can be used to permit the reparative mode (e.g., above 375 nm)to be processed separately or jointly with the tanning mode (e.g., 280-300 nm).
Figure 1 depicts an apparatus to mechanically provide discrete pulses of energy of selected wave length to effect tanning or DNA repair according to a preferred embodiment of the present invention;
Figure 2 is cross-section taken along line 2-2 of Figure 1, showing more detail of a modified version of the device shown in Figure 1;
Figure 3A is a schematic representation of a device to electronically produce discrete pulses of energy of selected wave length;
Figure 3B shows the device of Figure 3 with multiple electronic pulsed generators to produce uniform pulsed irradiation over a wide area;
Figures 4 and 5 depict alternate embodiments to mechanically produce discrete pulses of energy of selected wave length;
Figure 6 shows the electronic embodiment within a conventional tanning booth;
Figures 7A-7E depicts a representative wave form of discrete pulses generated by a mechanical pulse generator;
Figures 8A-8E depict representative wave forms of discrete pulses generated by an electronic pulse generator.
Figure 9 shows a perspective view of a radiation source inside a hollow cylinder having openings therethrough of one embodiment of the present invention;
Figure 10 shows a perspective view of a radiation source inside a pair of hollow cylinders having openings therethrough of another embodiment of the present invention;
Figure 11 shows selected radiation patterns for the apparatuses shown in Figures 9 and 10;
Figure 12 shows a top and bottom view of one embodiment of the present invention incorporating a plurality of radiation sources;
Figure 13 shows one means to axially rotate a plurality of single cylinders and another means to axially rotate a plurality of inner and outer cylinders in opposite directions, both means employing a motor, which could be used with the present invention; and,
Figure 14 shows alternative means to axially rotate cylinders using air turbine technology.

### DESCRIPTION OF PREFERRED EMBODIMENTS

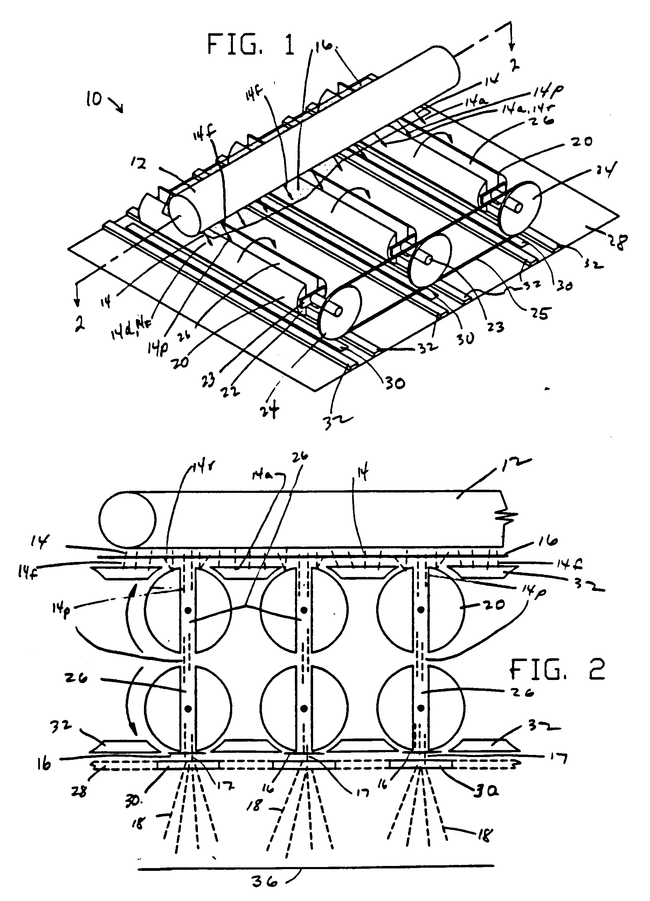

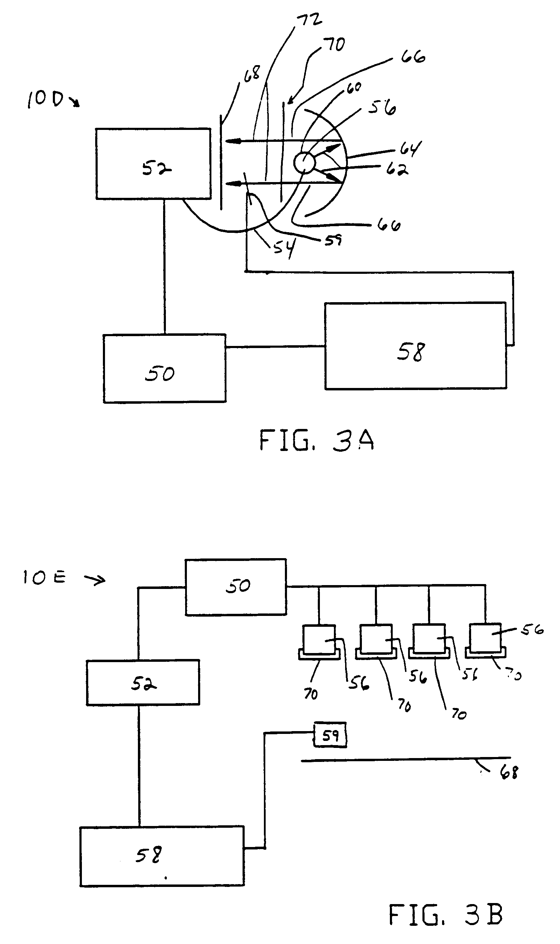

Two basic embodiments of the present invention to generate discrete pulses are presented: mechanical generators shown in Figures 1, 2, 4, 5, 7, 9 - 14; and electronic pulse generators shown in Figures 3, 6 and 8.

### Embodiment I: Mechanical Pulse Generation

Referring now to Figures 1 and 2, a mechanically-pulsed irradiation generation apparatus 10 is presented. A radiation source 12 is provided to generate radiation of first selected wave length 14 for tanning, within the range of 250 to 400 nm, preferably 280 to 300 nm and an apparent optimal wave length of about 290 nm. A second selected wave length between 320-600 nm may be utilized for DNA repair, preferably above 375 nm, with an apparent optimal wave length of about 460-500 nm and a target of 480 nm. The preferred or optimum wave length of radiation 14 may be selected by passing the radiation through one or more transmittance filters 16, which may be made of UVT plastic 260 (1/4" or approximately 6.4mm) available through EG&G Electro-Optics Division of EG&G, Inc., Salem Massachusetts, USA, or several other known materials, to produce filtered radiation of selected wave length 14f.

In Figure 1, unfiltered radiation 14 is generated by radiation source 12 above path blocking means, in this case rapidly rotating cylinders 20. Cylinders 20 are connected at at least one of their longitudinal ends 22 by connecting means 23 to rotating means 24, in this case sprockets 24 driven by chain means 25. Sprockets 24 and chain means 25 are driven by any suitable power means (not shown). Cylinders 20 are fitted with a slit 26 extending therethrough and extending the length of cylinders 20. It is seen that only when slit 26 of cylinders 20 are in proper alignment is energy 14p permitted to travel downward therethrough. When slits 26 are not in proper alignment, the path of filtered energy 14f is periodically blocked because it is either reflected off 14r or absorbed by 14a cylinders 20.

It is further seen that much of the time the path of energy 14 is blocked. Radiation is permitted to pass through slits 26 only for the brief discrete instant that slits 26 are in proper vertical alignment with radiation source 12. Otherwise, the path of energy is blocked by cylinders 20, and the energy is not passed into or emitted through slits 26. This results in discrete pulses of energy 17 being emitted from slits 26. A transmittance filter 16 may also be positioned at the exit end of slits 26, to provide discrete pulses of radiation of selected wave length 18. Selected wave length radiation 18 is then directed to the location 36 where the skin is to be effected, either tanned or DNA repaired. Preferably, when a plurality of cylinders is presented (to provide pulsed radiation over a wider area, for example), chain means 25 is of the timing chain variety, so that sprockets 24 may be rotated rapidly in synchronization with each other sprocket, thereby presenting slits 26 in synchronized alignment to provide uniform pulsed energy over the area.

The mechanical pulse generation apparatus of Figure 2 is provided with a filter block or grate 28 to prevent any radiation from passing therethrough and therebelow. Filter block 28 of Figure 1 is provided with radiation passing means in the form of slots 30 aligned with slits 26 of rotating cylinders 20, and those slots 30 extend through the thickness of filter block 28 to permit the pulsed radiation 17 to pass therethrough. To ensure that no unwanted radiation passes into slots 30, they are lined with blinders 32, made of any light absorbing material, such as closed cell foam or the like.

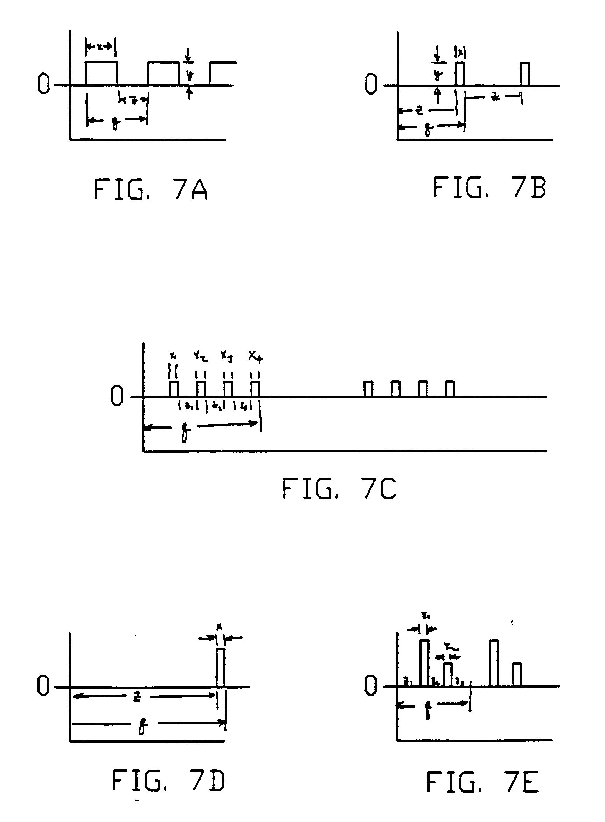

The mechanical pulse generation device of Figures 1 and 2 generates discrete pulses of selected energy having wave forms depicted in Figure 7. When the slits 26 are not fully aligned with slots 30 and radiation source generator 12, the path of the radiation is blocked and no radiation is emitted, represented by the "zero" line of the various graphs. As shown in Figure 7A, when slits 26, slots 30 and source 12 are in alignment, positive energy of strength or power level "y" is emitted for a pulse duration of "x". When rotation of cylinders 20 drives slots 30, slits 26 and light source 12 out of alignment, the unit "goes dark" and no energy is emitted for a period of "z". The duration of each pulse and the dark time between pulses depends upon the geometry of cylinders 20 and slits 26. For example, when cylinders 20 are one inch (2.54 cm) in diameter and slits 26 are 0.165" (approximately 4.2mm) wide, and they are rotated at 1000 revolutions per minute (rpm), the resulting discrete pulse of UV energy 17 emitted from the slots 30 of cylinder 20 is about 1.5 to 2.0 milliseconds.

Different arrangements of cylinders 20, slots 30 and slits 26 would produce different pulse characteristics. For example, Figure 7B shows two pulses of uniform duration and power, with a uniform dark time between each pulse. Figure 7C shows a series of uniform relatively low power pulses separated by a dark time duration relatively the same length as the series of pulses. Figure 7D shows a single pulse of higher power level after a relatively long dark period. Figure 7E shows a repeating variable multiple pulse sequence, with a first higher power pulse followed by a short dark period and then a lower power burst of about the same pulse duration, followed by a longer dark period until the pulse cycle repeats. The variety of pulse patterns within a particular cycle is limited only by the geometric imagination of the designer. For example, Figure 2 shows two rows of rotating cylinders 20, while Figure 1 depicts a single array of cylinders 20. Similarly, transmittance filter(s) 16 can be interchanged so that a single device is capable of emitting discrete pulses of radiation of two selected wave lengths, the first to promote tanning, and the second to promote DNA repair.

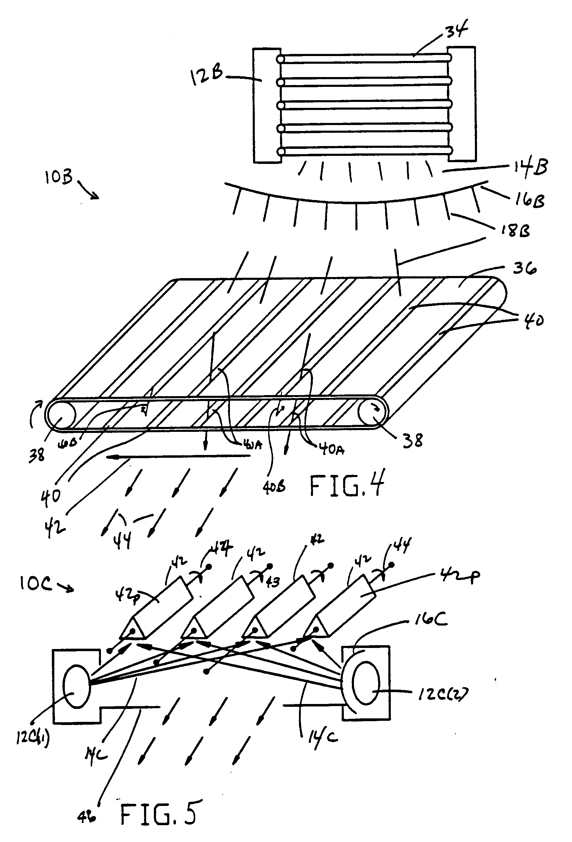

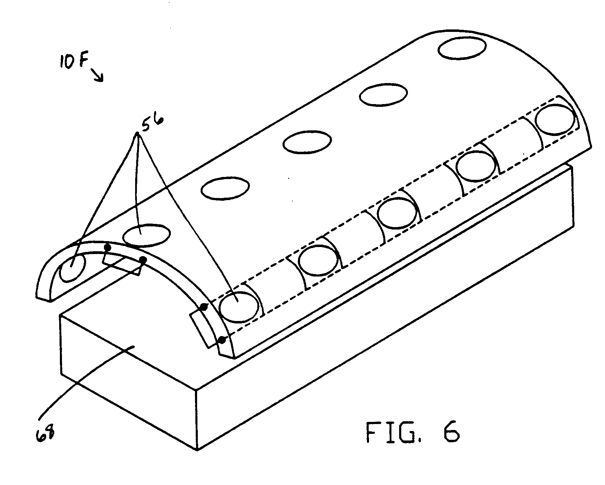

Referring to Figures 4 and 5, alternate embodiments of mechanically-pulsed generation devices are presented. In Figure 4, a pulse generator 10B is presented with a radiation source 12B for example, a series of fluorescent tubes emitting energy 14B of unfiltered or selected wave length. A transmittance filter 16B permits only selected wave length of energy 18B to pass there-through. Radiation source 12B and filter 16B are positioned above a flat panel 36 which is rotated about rollers 38 which are powered (not shown) to rapidly rotate. Flat panel 36 is provided with a plurality of parallel slats 41 which are generally impervious to the radiation and is further provided with a series of apertures 40, for example about 0.5" (1,27 cm) wide, through the thickness of panel 36.

As panel 36 is rotated about rollers 38 at about 1.5 feet per second (45.72 cm/sec), this causes upper slats 41 (and adjacent apertures 40) and lower slats 41 (and adjacent apertures 41) to contra-rotate with respect to each other. In this contra-rotating mode, the radiation path is blocked much of the time by the radiation-impervious slats 41; even when some radiation is permitted through a top aperture 40, most of the time it will be blocked 40B by a bottom radiation-impervious slat 41. However, from time to time upper and lower apertures 40 will be placed in alignment as shown by 40A, which permits filtered radiation 17 to pass completely through rotating panel 36, thereby presenting discrete pulses of emitted energy of about 1.5 milliseconds duration. A further filter 42 may be provided to further modify the selected wave length of the emitted pulses of radiation 44.

A device according to the embodiment depicted in Figure 4 could be presented out of doors with the source 12B being the sun. People could reside under device 10B and be subjected to only discrete pulses of energy from the sun, rather than being continuously exposed to the potentially damaging total array of UV energy normally presented by the sun. This could prevent skin damage in a major way.

In Figure 5, an apparatus 10C is presented where the discrete pulses of energy are provided by directing energy 14C from radiation sources 12C(1) and 12C(2) without filtering (12C(1)) or through transmittance filters 16C (from 12C(2)), onto one or more rotating reflecting surfaces 42 (e.g., mirrors). Reflecting surfaces 42 are presented in triangular or other discrete polyhedral form, and rotated 43 about their axes 44. It is seen that reflecting surfaces 42 are only capable of reflecting for the discrete period of time that a planar surface 42p is presented, thereby generating discrete reflected pulses of energy. This reflected radiation may be further filtered by a transmittance filter 46, if further selection of optimum wave length is desired.

It will be apparent to those skilled in the art that the emitted wave form of the emitted pulses from the devices of Figures 4 and 5 will be similar to those wave forms depicted in Figure 7. Again, a variety of pulse wave forms can be achieved, depending upon the geometric configuration(s) of the components.

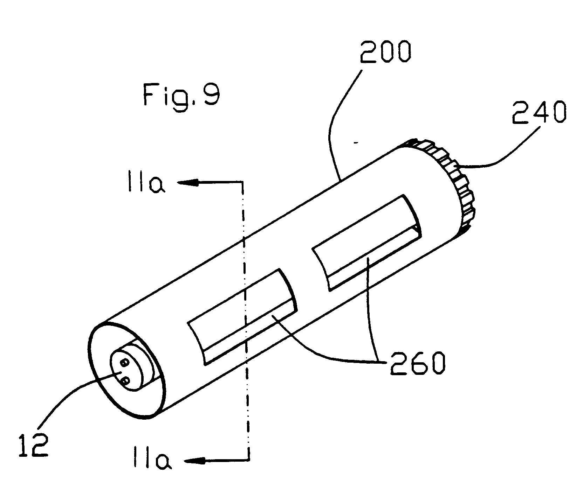

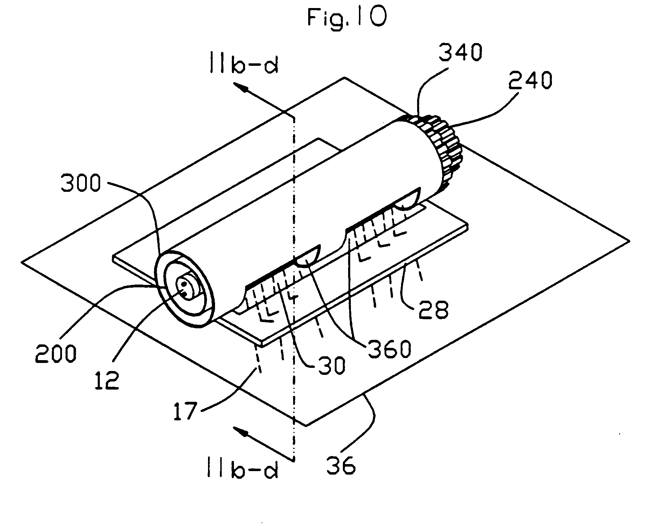

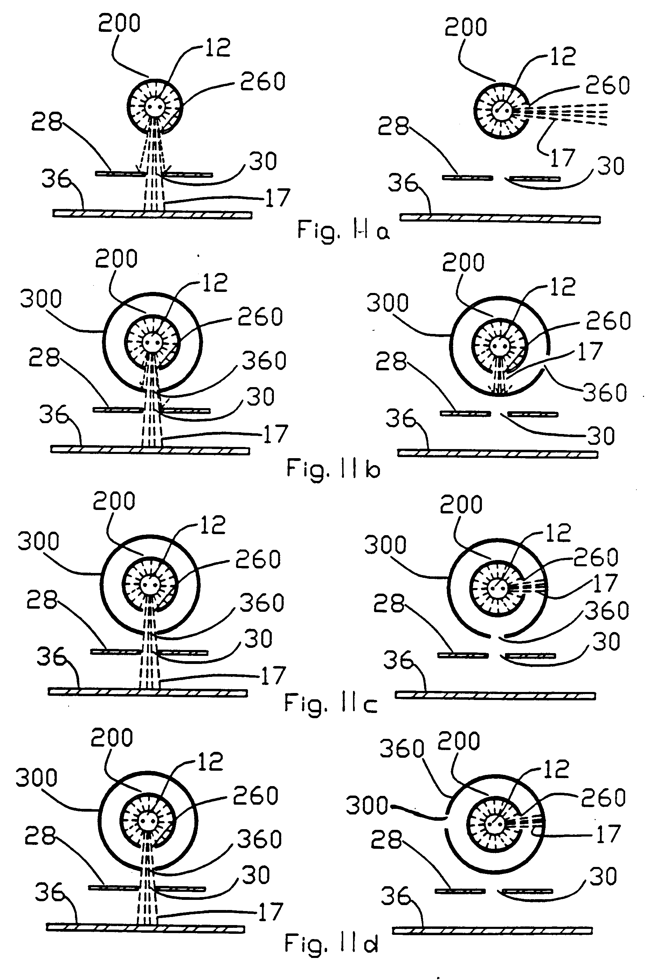

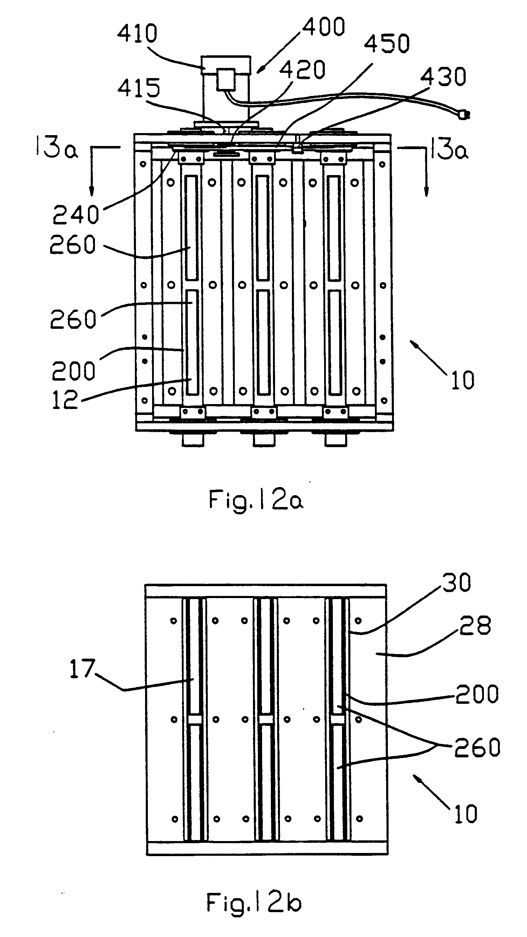

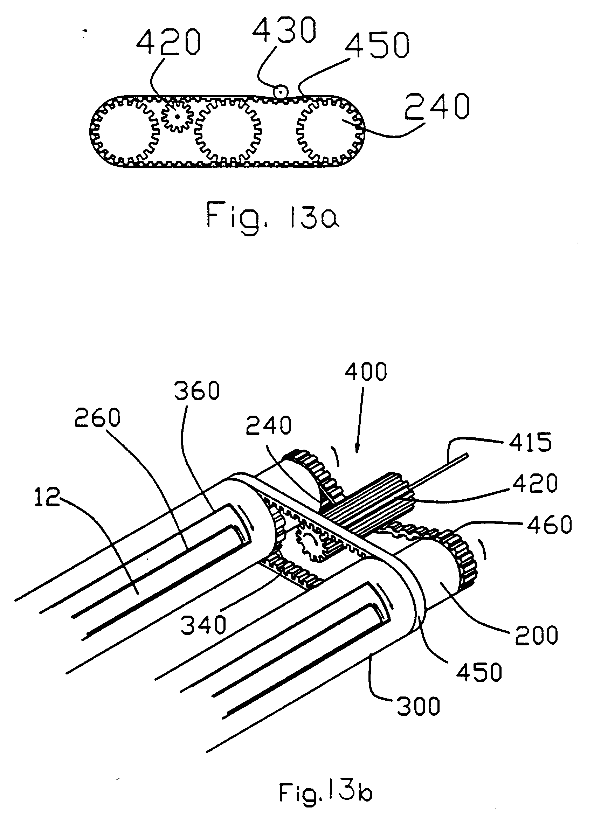

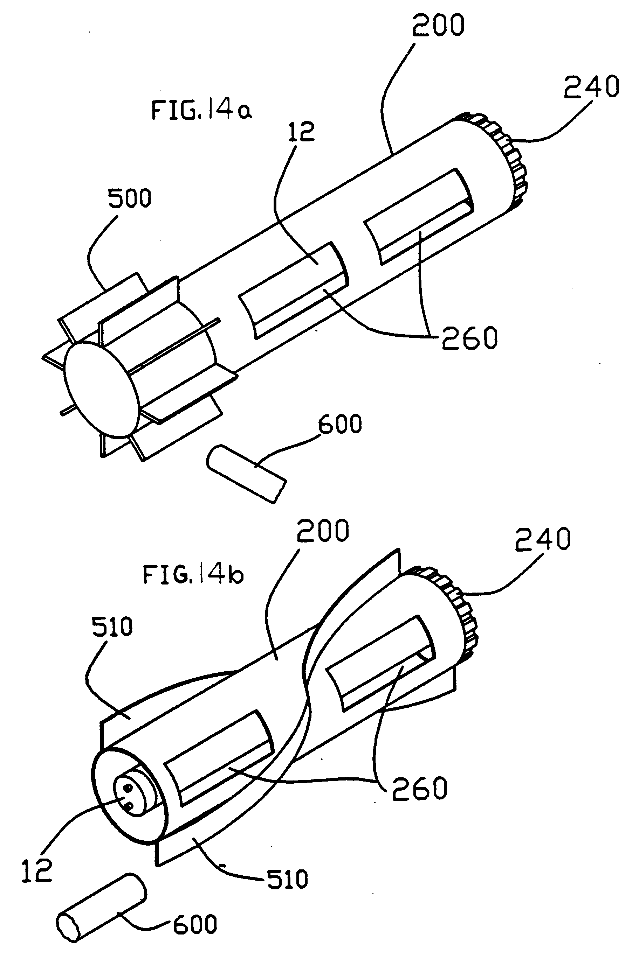

In Figures 1 and 2, cylinders 20 were adjacent to radiation source 12. Experience has shown that arrangement results in the majority of the radiation being wasted, as source 12 circumferentially radiates omnidirectionally and the slits 26 pass only that amount of radiation radiated toward them. Therefore, with reference to Figures 9 - 14, I show alternative embodiments which place cylinders having openings therethrough around the radiation sources and teach additional ways to rotate these cylinders. Figure 9 shows an circumferentially omnidirectional radiation source inside one hollow cylinder having openings therethrough of one embodiment of the present invention. Figure 10 shows an circumferentially omnidirectional radiation source inside a pair of hollow cylinders having openings therethrough of another embodiment of the present invention. Figure 11 shows selected radiation patterns for the apparatuses shown in Figures 9 and 10. Figure 12 shows a top and bottom view of one embodiment of the present invention incorporating a plurality of radiation sources, each inside one hollow cylinder. Figure 13 shows one means to axially rotate a plurality of single cylinders and another means to axially rotate a plurality of inner and outer cylinders in opposite directions which could be used with the present invention. Figure 14 shows how fins could be added to a cylinder to utilize air turbine technology to rotate the cylinder. For tanning and DNA repair, I believe that the inner and outer cylinder embodiment, such as shown in Figure 10, is preferable, as it is desirable to only radiate pulses in the direction(s) desired.

With reference now to Figure 9, a radiation source 12 is shown inside a first hollow cylinder 200 having two openings 260 in the cylindrical surface. As will be explained later, sprockets 240 located at one end of cylinder 200 will be used to rotate cylinder 200 about its axis. In Figure 10, the radiation source 12 and the first hollow cylinder 200 have been inserted into a second hollow cylinder 300. First cylinder 200 and second cylinder 300 are in coaxial alignment. The second cylinder 300 also has two openings 360 in its cylindrical surface. As shown in Figure 10, first hollow cylinder 200 has an axial length greater than that of second hollow cylinder 300. The end of cylinder 200 having sprockets 240 extends axially outside the end of cylinder 300 having sprockets 340. As will be explained later, sprockets 240 and 340 will be used to rotate cylinder 200 in one direction about its axis and cylinder 300 in the opposite direction about its axis.

Figure 10 also includes filter block or surface 28 having a slot or opening 30 therethrough. When radiation source 12, openings 260 in first cylinder 200, openings 360 in second cylinder 300, and opening 30 are in radiation communication, radiation from radiation source 12 passes therethrough. When one or both of first 200 and second 300 cylinders rotate, the alignment of openings 260, 360, and 30 is such that radiation passing through opening 30 from source 12 toward surface 36 is in the form of a pulse 17.

Figure 11a shows a two-dimensional cross-section along the lines 11a of Figure 9, with the addition of surface 28 having opening 30, surface 36, and radiation beam 17. With radiation source 12 continuously circumferentially radiating omnidirectionally, the rotation of cylinder 200 causes a "lighthouse effect." Radiation 17 passing through opening 260 will rotate around the axis of cylinder 200 causing a pulse of radiation to pass through opening 30 and irradiate a fixed location on surface 36 for each rotation of cylinder 200. However, for example, because of the rate of revolution which will be used for tanning, a human eye would not detect this "lighthouse effect", but, instead, would sense continuous radiation.

Increasing the reflectivity of the inner cylindrical surface of cylinder 200 for the wavelength(s) of radiation 17 being radiated by omnidirectional source 12 will cause more power per unit area to irradiate the fixed location on surface 36. In contrast, source 12 can be constructed so that it only radiates in the direction of the fixed location on surface 36 to be pulsed. There will then be no "lighthouse effect". Instead, rotating cylinder 200 will cause pulses of radiation to appear at the fixed location on surface 36 by having the inner cylindrical surface of cylinder 200 interrupt the radiation 17 radiating toward that fixed location. With this directional source 12, the inner cylindrical surface of cylinder 200 may be made more absorptive to the wavelength(s) of radiation 17 to decrease undesired reflections.

Figures 11b-d show two-dimensional cross-section views along the lines 11b-d of Figure 10. Figures 11b-d all show an circumferential omnidirectional radiating source 12, a first cylinder 200 having opening 260, a second cylinder 300 having opening 360, a surface 28 having opening 30, a surface 36, and a radiation beam 17. In Figure 11b, first cylinder 200 is stationary and second cylinder 300 rotates. In Figure 11c, first cylinder 200 rotates and second cylinder 300 is stationary. In Figure 11d, first cylinder 200 and second cylinder 300 rotate at the same number of revolutions per unit time, but in opposite directions. In all three of these configurations, one pulse 17 irradiates a location on surface 36 each time radiation source 12 and openings 260, 360, and 30 are in radiation communication. The rotational speed of the rotating one or both cylinders will determine the period "q". The geometric relationships between the size of the various openings and the cylinder dimensions, along with rotational speed will determine the length of time "x" when there is a pulse at surface 36. During the dark period "z" there is no pulse at surface 36. Therefore, the period is the sum of the pulse length and the dark period, represented as "$\text{x + z = q}$". For example, I envision that in tanning, the dark period will be at least three times longer than the pulse period. However, depending on the application and exposure desired, this relationship will vary greatly.

As was discussed with Figure 11a, increasing the reflectivity of the inner cylindrical surface of first cylinder 200 for the wavelength(s) of radiation 17 being radiated by circumferential omnidirectional source 12 will cause more power per unit area to irradiate the location on surface 36. Further, depending on the geometry, it may be desirable for the inner cylindrical surface of first cylinder 200 to have parabolic shape to geometrically focus radiation 17 through opening 260.

Because the only time a pulse is desired is when radiation source 12 and openings 260, 360, and 30 are in radiation communication, the outer cylindrical surface of first cylinder 200 and both the inner and outer cylindrical surfaces of second cylinder 300 can be made absorptive to the wavelength(s) of radiation 17 being radiated by source 12. Again, as was discussed with the single cylinder configuration of Figure 11a, source 12 can be constructed so that it only radiates in the direction where source 12 and openings 260, 360, and 30 are in radiation communication.

Figure 12a is a top view of an apparatus 10 to provide pulses of radiation. In operation, the top of apparatus 10 would have a protective cover installed. Apparatus 10 has three radiation sources 12 each contained in a hollow cylinder 200. Each cylinder 200 has two openings 260 through the cylindrical surface of cylinder 200. As shown, the two openings 260 in each cylinder 200 have a combined length which approximates the length of the radiation source 12 inside the cylinder 200. The openings 260 each have a width which approximates the diameter of the radiation source 12 inside the cylinder 200. Also, all openings 260 in all cylinders 200 are aligned in parallel, for example, all openings 260 are shown facing up. The three cylinders are shown spaced equally apart and parallel to each other. This spacing will be determined by how far away surface 36 to be irradiated by a pulse, shown in previous figures, is from apparatus 10 and the desired irradiation pattern on surface 36. For tanning and DNA repair, uniform power per unit area irradiation distribution is desired.

Figure 12b shows a bottom view of apparatus 10 of Figure 12a with cylinders 200 having been rotated 180 degrees from the position shown in Figure 12a so that openings 260 now all face the bottom of apparatus 10. At the instant shown in Figure 12b, a pulse of radiation 17 would be simultaneously transmitted from each radiation source 12 through openings 260 in each cylinder 200 and further through openings 30 in surface 28.

Figure 12a also shows one typical rotation means 400. Figure 13a shows a side view of means 400 along the lines 13a shown in Figure 12a. With reference to both Figure 12a and 13a, sprockets 240 at one end of each cylinder 200 are aligned in a plane. Means 400 is shown comprising a motor 410 having a shaft 415 connected to a sprocketed gear drive 420. A sprocketed endless conveyor 450 engages the appropriate sprockets 240 of each cylinder 200 and sprocketed gear drive 420. Conveyor tension means 430 maintains proper tension on sprocketed endless conveyor 450. As motor 410 rotates shaft 415 and thereby rotates sprocketed gear drive 420, sprocketed endless conveyor 450 rotates, thereby rotating cylinders 200.

Figure 13b shows how rotation means 400 could be used to rotate a pair of first hollow cylinders 200 axially in one direction and a pair of second hollow cylinders 300 axially in the opposite direction. Rotation means 400 comprises a motor (not shown) connected to shaft 415 which is connected to sprocketed gear drive 420. As with the three single cylinders 200 shown in Figures 12a and 13a, sprocketed endless conveyor 450 engages sprocketed gear drive 420. It also engages appropriate sprockets 340 of each second cylinder 300. A second sprocketed endless conveyor 460 having sprockets on both sides is used. Sprockets on one side of conveyor 460 engage appropriate sprockets 240 of each cylinder 200 and sprockets on the other side of conveyor 460 engage sprocketed gear drive 420. In this embodiment, conveyors 450 and 460 are sized to ensure proper rotational timing so that all cylinders 200 and all cylinders 300 rotate at the same number of revolutions per unit time. Openings 260 in all first cylinders 200 are in parallel, as are openings 360 in all second cylinders 300. This, along with the equal rotational speed of all cylinders 200 and 300 will ensure that radiation pulses 17 will simultaneously pass from each source 12 through openings 260 and 360 and will always be directed to the same location with each rotation of cylinders 200 and 300.

In the alternative, instead of connecting a motor to shaft 415, a means to employ air turbine technology could be connected. Simply by connecting to shaft 415 a device having a plurality of fins and by having a compressed air source provide high speed air onto these fins, shaft 415 would rotate as above. Conveyors 450 and 460 would again act as timing belts to control rotation of the cylinders 200 and 300.

Figures 14a and 14b show alternatives to this which also employ air turbine technology. In Figure 14a, fins 500 are added at the non-sprocketed end of cylinder 200 and compressed air is delivered onto fins 500 through nozzle 600 to rotate cylinder 200. Depending on the application, sprockets 240 can engage a conveyor, as previously described, to ensure that a plurality of cylinders will rotate with proper timing. Figure 14b incorporates fins 510 which helically wrap around the outside surface of cylinder 200, positioned so as to not interfere with the radiation exiting openings 260. Placing cylinder 200 inside cylinder 300, as previously disclosed, and placing nozzle 600 so that air is blown between the outer surface of cylinder 200 and the inner surface of cylinder 300 will cause cylinder 200 to rotate. Again, sprockets 240 can be used to ensure proper timing if a plurality of cylinders is employed.

For applications involving a cylinder 200 coaxially aligned with cylinder 300, such as was described in Figure 10, those skilled in the art can easily see how the fins 500 of Figure 14a could be placed on both cylinders 200 and 300 and how air could be directed to have the cylinders 200 and 300 rotate in opposite directions. Also, for the fins 510 of Figure 14b, the fins 510 of cylinder 200 and 510 of cylinder 300 would helix in opposite directions around the outside surface of their respective cylinders so that air would cause the cylinders to rotate in opposite directions. In this configuration, an outer sheath at least partway around the outside cylinder would be required to direct the air along the outside of this outside cylinder to cause it to rotate. The outside cylinder performs this "sheath" function for the inner cylinder.

Embodiment II: Stroboscopic Pulse Generation An electronic pulse generator 10D, utilizing stroboscopic flash tubes, is shown in Figures 3A and 3B. In Figure 3A, the pulse generator 10D is presented with a power supply 50 linked to a lamp trigger to provide timed pulses or flashes of flashlamp 56 via connection 54. The duration and frequency of the pulses is controlled by a signal processing timing means 58 and signal detector means 59. Flashlamp devices (sometimes called flashtubes) are known for other unrelated applications and are available from, for example, EG&G Electro-Optics Division of EG&G, Inc. of Salem, Massachusetts, USA. EG&G has published several technical brochures and operating manuals for its flashlamps (e.g., Short-Arc Xenon Flashlamps and Power Supplies, Data Sheet F1022B-1 [3/88]; Flashlamp Applications Manual [4/88]). Continuous UV lamps for the mechanical pulse generators can be purchased from Southern New England Ultraviolet Co., Hamden, CT, USA, such as their model nos. RPR3500 (peak near 350 nm; 1/6 peak = 320 nm; 4.5 watts) and RPR3000A (peak near 300 nm; 1/6 peak = 270 nm; 15 watts).

In the device of Figure 3A, by selecting the type of flashlamp 56 (e.g., a xenon flashlamp) and the glass envelope 60 surrounding flashlamp 56, the spectral output 62 of flashlamp 56 can be controlled to provide radiation pulses of a selected wave length (e.g., a xenon flashtube will have an emission of UV irradiation of one general spectrum with most of the emission at a wave length of about 250-300 nm, while a krypton flashlamp will have a different spectral emission. In addition, the selection of the glass envelope 60 surrounding flashlamp 56 will also have an impact on the spectral emission of radiation output 62. EG&G provides several different types of flashlamps and glass envelopes, which may be selected to achieve the desired spectral characteristics. For further details, see the aforementioned EG&G publications.

Pulsed flashlamp output 62 will typically (but not necessarily) be imposed upon a reflector surface 64, for example an aluminized reflector, which will reflect the pulses 66 toward the object to be irradiated 68. A transmittance filter 70 composed for example of UVT plastic may be interposed between reflector 64 and target location 68 to further filter the radiation pulses to a more narrowly selected wave length. For example, an EG&G xenon strobe or flash lamp [with UV Glass Corning 9823] coupled with a 10 nm band width filter can selectively provide 285-295 nm light with pulse duration ranging from 10-100 microseconds. If desired, filter 70 can be placed closer in proximity to flashlamp 56, as shown in Figure 3B.

I believe that maximum tanning in human skin will occur at a UV irradiation wave length of about 280 to 300 nm, preferably about 290 nm. By utilizing appropriate combinations of flashlamp 56 plus glass envelope 60 plus reflector surface 64 plus filter 70, the resulting filtered pulses of UV irradiation can be controlled to that selected wave length. Similarly, I believe that maximum DNA repair occurs at a wave length above 375 nm, preferably between 480 and 500 nm, optimally about 490 nm. Choosing a second appropriate combination of flashlamp 56 plus glass envelope 60 plus reflector 64 plus filter 70 can produce pulsed irradiation of this second desired frequency for optimal DNA repair. If desired, flashlamps can be arrayed in such a manner that a single apparatus could provide irradiation of two selected wave lengths, the first to promote tanning, and the second to promote DNA repair.

In apparatus 10E shown in Figure 3B, it is seen that multiple flashlamps 56 can be arrayed to provide pulsed irradiation of selected frequency over a wider area. In Figure 6, multiple flashlamps 56 are shown arrayed in a standard tanning booth 10F, which can irradiate a wide enough target location 68 with pulsed irradiation of selected wave length to promote tanning (or DNA repair) in an adult human.

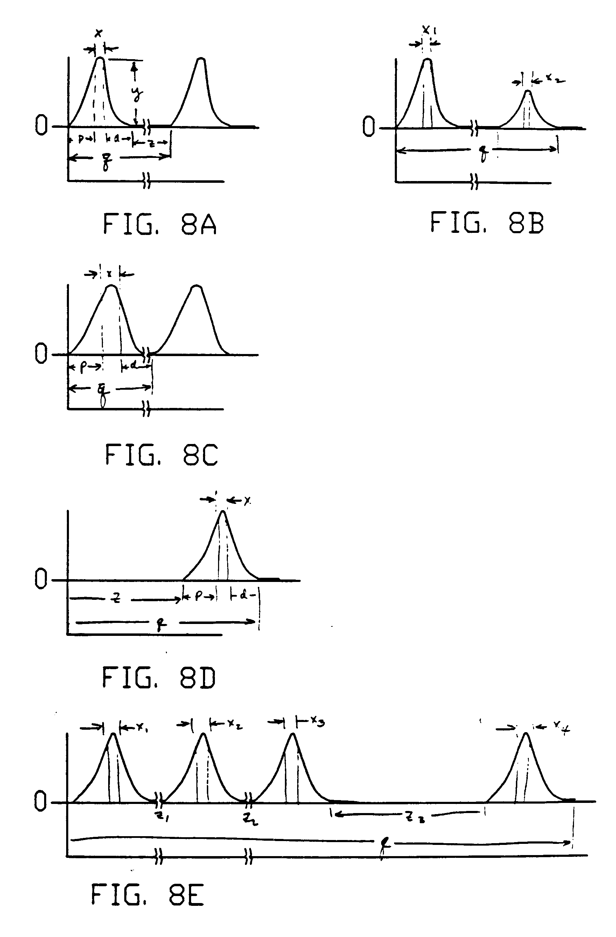

Referring now to Figure 8, it is seen that the wave form of pulses provided electronically or stroboscopically is different than the wave form of mechanically produced pulses (Figure 7). Typically, within each energy cycle q, there is a rapid but not instantaneous energization toward a peak power or strength y, which only lasts for a short burst of time x. Then the pulse wave form rapidly decays towards a zero energy state. It is difficult to accurately portray the characteristics of an electronically produced pulse, because the time axis is so disproportionate. For example,in a total cycle time of q (say, one second) a typical xenon flashlamp available from EG&G will provide a total pulse duration of about 50 microseconds, leaving a dark portion of 999,950 microseconds; this ratio is impossible to show on a uniform time axis. Further, only a portion ("x") of that 50 microseconds will be at peak power ("y"), with the balance of the time utilized to bring the flashlamp rapidly up to peak ("p"), and then rapidly decaying to negligible ("d").

Referring to Figures 8B-8E, as with the mechanical pulse generator, a wide variety of electronically-generated cycle formats is available. Figure 8B shows a cycle containing two pulses, with the first pulse being of greater power than the second. In Figure 8C, a waveform with a longer time at peak is shown. In Figure 8D, a single pulse preceded by a long dark period is shown. In Figure 8E, a cycle is shown which contains three uniform bursts followed by a long dark period and concluding with a fourth pulse. The potential combinations of cycle formats and wave forms is virtually unlimited. Of course, the actual wave forms are quite variable, and reference should be had to the EG&G manual for more precise descriptions.

Referring again to Figures 7 and 8, the key to this invention is to provide, within each cycle ("q") of energy input, a "dark period" ("z") where negligible energy is impressed upon the skin. Energy exposure, either to the frequency selected for tanning-inducing irradiation (280-400 nm) or to the frequency selected for DNA repair irradiation (350-600 nm), is provided for only in very short, discrete pulses, which fill only a very short time portion ("x") of the total energy cycle ($\text{q = x + z}$), as shown in the various wave forms of Figures 7 and 8. For example, in a one-second energy cycle, an electronic embodiment of the present invention (e.g., the apparatus depicted and described in Figures 3A/3B) would provide one pulse per second of UV energy of "y" power or strength for a duration "x", say 50 microseconds. For a pulse duration of 50 microseconds, the skin is exposed to energy for only 0.005% of the energy cycle; 99.995% of the energy cycle is "dark" time of negligible energy exposure.

A mechanical pulse generator as described above in Figure 1 would produce a two millisecond pulse in each one second cycle, resulting in an energy cycle that would be 99.8% dark, and energized only 0.2% of the total cycle time. Electronic/ stroboscopic pulse generators are available which could produce pulses in the range of 1 picosecond (0.000000001 sec), which could be of a very high power level to induce either tanning or DNA repair with a single high power pulse, and leave the vast majority of the energy cycle as "dark" time (i.e., 99.9999999% dark). While the extreme proportions of the dark time may have greatest salutary effect on enhancing the cellular reparative process(es), it is expected that for some events, only a brief dark time will be sufficient to produce the desired result, say five to ten percent of the cycle time.

In Figure 7C, a cycle containing four discrete pulses is shown. If the total cycle duration q is one second and each of these pulses is of 50 microseconds duration, the total energized portion of each one second cycle is 200 microseconds or 0.2 milliseconds. The total dark time associated with such a four-pulse cycle is therefore 99.98%; each cycle is only energized 0.02% of the time. Even when each pulse is a relatively long two milliseconds within a one second total cycle duration, the total energized portion of each cycle is only 0.2%; 99.8% of the cycle time is "dark" time. It is apparent that every embodiment of the present invention produces a total energy cycle wherein the vast majority of the total energy cycle time is "dark" time, thereby vastly reducing total cumulative energy exposure. Compared to continuous energization for the current state of the art tanning apparati, the present invention produces an energy cycle that is enormously less energy invasive, yet still produces the desired effect (either tanning or DNA repair).

While the principles of this invention have been presented in illustrative embodiments, there are potentially many modifications of the radiation source (excimer lasers, for example, are capable of producing as many as 10,000 discrete pulses per second, each pulse having a duration on the order of picoseconds), mechanical modifications (belt technology for example), and gases used for the stroboscopic applications.

## Claims

1. Apparatus for tanning skin comprising:
means (36) defining a location at which the skin is to be positioned for tanning;
at least one electromagnetic pulse generating means for directing discrete pulses of electromagnetic radiation towards said location defining means (36) for a plurality of pulse cycle periods having a total cycle time (q) which includes a dark time (z) during which the skin is exposed to negligible radiation energy, said dark time (z) being at least one half of said total cycle time (q), said total cycle time (q) for each of said plurality of pulse cycle periods having an equal time duration, each of said plurality of pulse cycle periods including at least one discrete pulse (x1,x2,x3,x4) of electromagnetic radiation, wherein
a) the at least one electromagnetic pulse generating means generates radiation selected from at least one wave length of between 250 and 400 nanometers for a selected number of pulse cycle periods per second, and
b) each said discrete pulse (x1...x4) has a pulse duration time of from about 1 picosecond to about 20 milliseconds.

2. An apparatus as recited in claim 1, said electromagnetic pulse generating means including continuous electromagnetic generating means (12,12B,12C) for continuously emitting electromagnetic radiation in a path towards a location defining means (36) at which the skin is to be positioned for tanning and path blocking means (20,41,200,300) for periodically blocking the path of the continuously emitted electromagnetic radiation to obtain the discrete pulses (x1...x4) of electromagnetic radiation.

3. An apparatus as recited in claim 2, said path blocking means (20,41,200,300) comprising a plurality of parallel rotating slats (41) located between said location defining means (36) and said continuous electromagnetic generating means (12B), each of the plurality of parallel rotating slats having an aperture (40) therethrough to allow the passage of the continuously emitted electromagnetic radiation at a predetermined rotational position.

4. An apparatus as recited on claim 2, said path blocking means (20,41,200,300) comprising one or more rotating cylinders (20) located between said location defining means (36) and said continuously electromagnetic generating means, (12,12B,12C), each cylinder (20) having an aperture (26) therethrough to allow the passage of the continuously emitted electromagnetic radiation at a predetermined rotational position.

5. An apparatus as recited in claim 2, said path blocking means (20,41,200,300) comprising one or more rotating hollow cylinders (200) located around said continuous electromagnetic generating means (12), each cylinder (200) having at least one aperture (260) therethrough to allow the passage of the continuously emitted electromagnetic radiation at a predetermined rotational position toward said location defining means (36).

6. An apparatus as recited in claim 2, said path blocking means comprising a plurality of parallel rotatable members (200,300) and means (240,340) for rotating said plurality of parallel rotatable members (200,300) each of the plurality of rotatable members having at least one reflective surface for, when said rotatable member is rotated, periodically reflecting a continuously emitted electromagnetic radiation towards said location defining means (36).

7. An apparatus as recited in claim 1, said electromagnetic pulse generating means including at least one strobe light (56) to intermittently produce electromagnetic irradiation.

8. An apparatus as recited in claim 7, said electromagnetic pulse generating means (56,70) further comprising a filter means (70) located between said at least one strobe light (56) and a location defining means (36) at which the skin is to be positioned for tanning for, passing therethrough intermittent electromagnetic radiation of one or more selected wave length bands.

9. An apparatus as recited in any preceding claim, said electromagnetic pulse generating means (56,70) comprising a first means (56) for emitting electromagnetic radiation of said first selected wave length to induce tanning , and a second means (56) for emitting electromagnetic radiation of said second selected wave length to effect repairing ultraviolet radiation induced DNA damage within skin cells.

10. An apparatus as recited in any preceding claim, wherein said at least one electromagnetic pulse generating means (12,12B,12C,56,70) for directing discrete pulses of electromagnetic radiation towards said location defining means (36) includes at least two electromagnetic pulse generating means (12,...70) and wherein said discrete pulses directed towards said location defining means (36) are synchronized.

11. An apparatus as recited in any preceding claim, further comprising:
at least one filter means (16,70) located at a location between said electromagnetic pulse generating means (12,...70) and a skin location means (36), whereby only one electromagnetic radiation of one or more desired wave lengths is passed therethrough.

12. An apparatus as recited in any preceding claim , wherein said at least one electromagnetic pulse generating means (12,...70) generates electromagnetic radiation of one or more preselected desired wave lengths.

13. An apparatus as recited in claim 9 or in any of the claims 10-12 when dependent on claim 9, wherein the discrete pulses (x1,...x4) of radiation for repairing ultraviolet radiation-induced DNA damage within skin cells have a wave length of about 320 to 600 nanometers.

14. An apparatus as recited in any preceding claim, wherein the discrete pulses (x1,...x4) of radiation for tanning have a wave length of about 280 to 300 nanometers.

15. An apparatus as recited in any preceding claim, wherein the discrete pulses (x1,...x4) of radiation for tanning have a wave length of about 290 nanometers.

16. An apparatus as recited in claim 9 or in any of the claims 10-15 when dependent on claim 9, wherein the discrete pulses of radiation for repairing ultraviolet radiation-induced DNA damage within skin cells have a wave length of about 460 to 500 nanometers.

17. An apparatus as recited in claim 9 or in any of the claims 10-16 when dependent on claim 9, wherein the discrete pulses (x1,...x4) of radiation for repairing ultraviolet radiation-induced DNA damage within skin cells have a wave length of about 480 nanometers.

## Patentansprüche

1. Gerät zum Bräunen von Haut mit:
einer einen Ort zum Anordnen der zu bräunenden Haut definierenden Einrichtung (36);
mindestens einer Einrichtung zum Erzeugen von elektromagnetischen Impulsen zum Richten von diskreten Impulsen elektromagnetischer Strahlung zu der den Ort definierenden Einrichtung (36) für eine Vielzahl von Impulszyklen-Perioden, die eine Gesamtzykluszeitdauer (q) haben, die eine Dunkelzeit (z) umfaßt, während der die Haut einer vernachlässigbaren Strahlungsenergie ausgesetzt ist, wobei die Dunkelzeit (z) mindestens die Hälfte der Gesamtzykluszeitdauer (q) beträgt, die Gesamtzykluszeitdauer (q) für jede der Vielzahl von Impulszyklen-Perioden eine gleiche Zeitdauer hat und jede der Vielzahl von Impulszyklen-Perioden mindestens einen diskreten Impuls (x1, x2, x3, x4) der elektromagnetischen Strahlung umfaßt, wobei
a) die mindestens eine Einrichtung zum Erzeugen von elektromagnetischen Impulsen eine Strahlung erzeugt, die aus mindestens einer Wellenlänge zwischen 250 und 400 Nanometern für eine ausgewählte Anzahl von Impulszyklen-Perioden pro Sekunde ausgewählt ist, und
b) jeder der diskreten Impulse (x1,....x4) eine Impulsdauer von etwa einer Pikosekunde bis etwa 20 Millisekunden hat.

2. Gerät nach Anspruch 1, bei dem die die elektromagnetischen Impulse erzeugende Einrichtung eine Einrichtung (12, 12B, 12C) zum kontinuierlichen Abstrahlen elektromagnetischer Strahlung in einem Pfad zu der den Ort definierenden Einrichtung (36), an dem die zu bräunende Haut anzuordnen ist, und eine den Pfad blockierende Einrichtung (20, 41, 200, 300) zum periodischen Blockieren des Pfades der kontinuierlich abgestrahlten elektromagnetischen Strahlung umfaßt, um die diskreten Impulse (x1...x4) der elektromagnetischen Strahlung zu erhalten.

3. Gerät nach Anspruch 2, bei dem die Einrichtung (20, 41, 200, 300) zum Blockieren des Pfades eine Vielzahl von parallelen, rotierenden Lamellen (41) aufweist, die zwischen der den Ort definierenden Einrichtung (36) und der Einrichtung (12B) zum Erzeugen der kontinuierlichen elektromagnetischen Strahlung angeordnet sind, wobei jede der Vielzahl von parallelen, rotierenden Lamellen eine Öffnung (40) durch sie hindurch haben, um das Hindurchtreten der kontinuierlich emittierten elektromagnetischen Strahlung bei einer bestimmten Drehstellung zu ermöglichen.

4. Gerät nach Anspruch 2, bei dem die Einrichtung (20, 41, 200, 300) zum Blockieren des Pfades einen oder mehrere rotierende Zylinder (20) aufweist, die zwischen der den Ort definierenden Einrichtung (36) und der Einrichtung (12, 12B, 12C) zum kontinuierlichen Erzeugen der elektromagnetischen Strahlung angeordnet sind, wobei jeder Zylinder (20) eine Öffnung (26) hat, durch die die kontinuierlich erzeugte elektromagnetische Strahlung bei einer bestimmten Drehstellung hindurchtreten kann.

5. Gerät nach Anspruch 2, bei dem die Einrichtung (20, 41, 200, 300) zum Blockieren des Pfades einen oder mehrere rotierende Hohlzylinder (200) aufweist, die um die Einrichtung (12) zum Erzeugen der kontinuierlichen elektromagnetischen Strahlung herum angeordnet sind, wobei jeder Zylinder (200) mindestens eine Öffnung (260) hat, durch die die kontinuierlich emittierte elektromagnetische Strahlung bei einer bestimmten Drehstellung zu der den Ort definierenden Einrichtung (36) hindurchtreten kann.

6. Gerät nach Anspruch 2, bei dem die Einrichtung zum Blockieren des Pfades eine Vielzahl von parallelen, drehbaren Teilen (200, 300) und eine Einrichtung (240, 340) zum Drehen der Vielzahl von parallelen drehbaren Teilen (200, 300) aufweist, wobei jedes der Vielzahl von drehbaren Teilen mindestens eine reflektierende Oberfläche hat, die, wenn das drehbare Teil gedreht wird, periodisch die kontinuierlich ermittierte elektromagnetische Strahlung zu der den Ort definierenden Einrichtung (36) hin reflektiert.

7. Gerät nach Anspruch 1, bei dem die Einrichtung zum Erzeugen der elektromagnetischen Impulse mindestens eine Stroboskoplichtquelle (56) umfaßt, die intermittierend elektromagnetische Strahlung erzeugt.

8. Gerät nach Anspruch 7, bei dem die Einrichtung (56, 70) zum Erzeugen der elektromagnetischen Impulse weiterhin eine Filtereinrichtung (70) aufweist, die zwischen der mindestens einen stroboskopischen Lichtquelle (56) und der den Ort definierenden Einrichtung (36) angeordnet ist, an dem die zu bräunende Haut anzuordnen ist, um durch die Filtereinrichtung hindurch intermittierende elektromagnetische Strahlung einer oder mehrerer ausgewählter Wellenlängenbänder hindurchzulassen.

9. Gerät nach einem der vorhergehenden Ansprüche, bei dem die Einrichtung (56, 70) zum Erzeugen der elektromagnetischen Impulse eine erste Einrichtung (56) zum Emittieren elektromagnetischer Strahlung der ersten ausgewählten Wellenlänge zum Bewirken von Bräunung und eine zweite Einrichtung (56) zum Emittieren elektromagnetischer Strahlung der zweiten ausgewählten Wellenlänge aufweist, um eine Heilung einer durch Ultraviolettstrahlung bewirkten DNA-Schädigung innerhalb von Hautzellen zu bewirken.

10. Gerät nach einem der vorhergehenden Ansprüche, wobei die mindestens eine Einrichtung (12, 12B, 12C, 56, 70) zum Erzeugen der elektromagnetischen Impulse zum Richten von diskreten Impulsen der elektromagnetischen Strahlung zu der den Ort definierenden Einrichtung (36) mindestens zwei Einrichtungen (12,...70) zum Erzeugen von elektromagnetischen Impulsen umfaßt und wobei die zu der den Ort definierenden Einrichtung (36) gerichteten diskreten Impulse synchronisiert sind.

11. Gerät nach einem der vorhergehenden Ansprüche, das weiterhin aufweist:
mindestens eine Filtereinrichtung (16, 70), die an einem Ort zwischen der Einrichtung (12,...70) zum Erzeugen der elektromagnetischen Impulse und der den Ort für die Haut definierenden Einrichtung (36) angeordnet ist, wodurch nur eine elektromagnetische Strahlung von einer oder mehreren gewünschten Wellenlängen durch die Filtereinrichtung hindurchgelangt.

12. Gerät nach einem der vorhergehenden Ansprüche, wobei mindestens eine Einrichtung (12,...70) zum Erzeugen der elektromagnetischen Impulse eine elektromagnetische Strahlung einer oder mehrerer vorgewählter gewünschter Wellenlängen erzeugt.

13. Gerät nach Anspruch 9 oder einem der Ansprüche 10 bis 12, sofern diese von Anspruch 9 abhängig sind, wobei die diskreten Impulse (x1,...x4) der Strahlung zum Heilen einer durch Ultraviolettstrahlung bewirkten DNA-Schädigung innerhalb der Hautzellen eine Wellenlänge von etwa 320 bis 600 Nanometern hat.

14. Gerät nach einem der vorhergehenden Ansprüche, wobei die diskreten Impulse (x1,...x4) der Strahlung zum Bräunen eine Wellenlänge von etwa 280 bis 300 Nanometern hat.

15. Gerät nach einem der vorhergehenden Ansprüche, wobei die diskreten Impulse (x1,...x4) der Strahlung zum Bräunen eine Wellenlänge von etwa 290 Nanometern hat.

16. Gerät nach Anspruch 9 oder nach einem der Ansprüche 10 bis 15, sofern diese von Anspruch 9 abhängig sind, wobei die diskreten Impulse der Strahlung zur Heilung einer durch Ultraviolettstrahlung bewirkten DNA-Schädigung innerhalb der Hautzellen eine Wellenlänge von etwa 460 bis 500 Nanometern hat.

17. Gerät nach Anspruch 9 oder einem der Ansprüche 10 bis 16, sofern diese von Anspruch 9 abhängig sind, wobei die diskreten Impulse (x1,...x4) der Strahlung zum Heilen einer durch Ultraviolettstrahlung bewirkten DNA-Schädigung innerhalb der Hautzellen eine Wellenlänge von etwa 480 Nanometern hat.

## Revendications

1. Dispositif pour le bronzage de la peau comportant :
des moyens (36) définissant un emplacement au niveau duquel la peau doit être positionnée pour bronzer ;
au moins des premiers moyens de production d'impulsions électromagnétiques pour diriger des impulsions discrètes d'un rayonnement électromagnétique vers lesdits moyens définissant un emplacement (36) pendant plusieurs périodes de cycle d'impulsions ayant un temps total de cycle (q) qui comporte un temps sombre (z) pendant lequel la peau est exposée a une énergie de rayonnement négligeable, ledit temps sombre (z) étant égal au moins à la moitié dudit temps total de cycle (q), ledit temps total de cycle (q) pour chacune desdites plusieurs périodes de cycle d'impulsions ayant une durée de temps égale, chacune desdites plusieurs périodes de cycle d'impulsions comportant au moins une impulsion discrète (x1, x2, x3, x4) d'un rayonnement électromagnétique, dans lequel
a) les au moins des premiers moyens de production d'impulsions électromagnétiques produisent un rayonnement sélectionné à partir d'au moins une longueur d'onde située entre 250 et 400 nanomètres pour un nombre sélectionné de périodes de cycle d'impulsions par seconde et,
b) chaque dite impulsion discrète (x1 ... x4) a un temps de durée d'impulsion allant d'environ 1 picoseconde à environ 20 millisecondes.

2. Dispositif selon la revendication 1, dans lequel lesdits moyens de production d'impulsions électromagnétiques comportent des moyens de production de rayonnement électromagnétique continu (12, 12b, 12c) pour émettre en continu un rayonnement électromagnétique dans un trajet en direction des moyens définissant un emplacement (36) au niveau duquel la peau doit être positionnée pour le bronzage et des moyens de blocage de trajet (20, 41, 200, 300) pour bloquer périodiquement le trajet du rayonnement électromagnétique émis en continu pour obtenir les impulsions discrètes (x1 ... x4) du rayonnement électromagnétique.

3. Dispositif selon la revendication 2, dans lequel lesdits moyens de blocage de trajet (20, 41, 200, 300) comportent plusieurs lattes rotatives parallèles (41) situées entre lesdits moyens définissant une emplacement (36) et lesdits moyens de production de rayonnement électromagnétique continu (12B), chacune des plusieurs lattes rotatives parallèles ayant une ouverture (40) traversante pour permettre le passage du rayonnement électromagnétique émis en continu à une position de rotation prédéterminée.

4. Dispositif selon la revendication 2, dans lequel lesdits moyens de blocage de trajet (20, 41, 200, 300) comportent un ou plusieurs cylindres rotatifs (20) situés entre lesdits moyens définissant un emplacement (36) et lesdits moyens de production de rayonnement électromagnétique en continu (12, 12B, 12C), chaque cylindre (20) ayant une ouverture traversante (26) pour permettre le passage du rayonnement électromagnétique émis en continu à une position de rotation prédéterminée.

5. Dispositif selon la revendication 2, dans lequel lesdits moyens de blocage de trajet (20, 41, 200, 300) comportent un ou plusieurs cylindres creux rotatifs (200) situés autour desdits moyens de production de rayonnement électromagnétique continu (12), chaque cylindre (200) ayant au moins une ouverture traversante (260) pour permettre le passage du rayonnement électromagnétique émis en continu à une position de rotation prédéterminée en direction desdits moyens définissant un emplacement (36).

6. Dispositif selon la revendication 2, dans lequel lesdits moyens de blocage de trajet comportent plusieurs éléments rotatifs parallèles (200, 300) et des moyens (240, 340) pour mettre en rotation lesdits plusieurs éléments rotatifs parallèles (200, 300), chacun des plusieurs éléments rotatifs ayant au moins une surface de réflexion pour, lorsque ledit élément rotatif est mis en rotation, réfléchir périodiquement un rayonnement électromagnétique émis en continu en direction desdits moyens définissant un emplacement (36).

7. Dispositif selon la revendication 1, dans lequel lesdits moyens de production d'impulsions électromagnétiques comportent au moins une lumière stroboscopique (56) pour produire de manière intermittente un rayonnement électromagnétique.

8. Dispositif selon la revendication 7, dans lequel lesdits moyens de production d'impulsions électromagnétiques (56, 70) comportent de plus des moyens filtrants (70) situés entre ladite au moins une lumière stroboscopique (56) et des moyens définissant un emplacement (36) au niveau duquel la peau doit être positionnée pour le bronzage, pour faire passer à travers ceux-ci un rayonnement électromagnétique intermittent ayant une ou plusieurs bandes de longueurs d'onde sélectionnées.

9. Dispositif selon l'une quelconque des revendications précédentes, dans lequel lesdits moyens de production d'impulsions électromagnétiques (56, 70) comportent des premiers moyens (56) pour émettre un rayonnement électromagnétique ayant ladite première longueur d'onde sélectionnée pour provoquer le bronzage, et des seconds moyens (56) pour émettre un rayonnement électromagnétique ayant ladite seconde longueur d'onde sélectionnée pour effectuer la réparation d'une lésion d'ADN provoquée par un rayonnement ultraviolet dans les cellules de la peau.

10. Dispositif selon l'une quelconque des revendications précédentes, dans lequel lesdits au moins premiers moyens de production d'impulsions électromagnétiques (12, 12B, 12C, 56, 70) pour diriger des impulsions discrètes d'un rayonnement électromagnétique en direction desdits moyens définissant un emplacement (36), comportent au moins deux moyens de production d'impulsions électromagnétiques (12, ... 70) et dans lequel lesdites impulsions discrètes dirigées en direction desdits moyens définissant un emplacement (36) sont synchronisées.

11. Dispositif selon l'une quelconque des revendications précédentes, comportant de plus :
au moins des premiers moyens filtrants (16, 70) situés à un emplacement entre lesdits moyens de production d'impulsions électromagnétiques (12, ..., 70), et des moyens (36) de positionnement de peau, de sorte que seul un rayonnement électromagnétique ayant une ou plusieurs longueurs d'ondes voulues passent à travers ceux-ci.

12. Dispositif selon l'une quelconque des revendications précédentes, dans lequel lesdits au moins premiers moyens de production d'impulsions électromagnétiques (12, ... 70), produisent un rayonnement électromagnétique ayant une ou plusieurs longueurs d'onde voulues présélectionnées.

13. Dispositif selon la revendication 9 ou selon l'une quelconque des revendications 10 à 12 lorsqu'elles dépendent de la revendication 9, dans lequel les impulsions discrètes (x1, ... x4) d'un rayonnement pour réparer une lésion d'ADN provoquée par un rayonnement ultraviolet dans les cellules de la peau ont une longueur d'onde d'environ 320 à 600 nanomètres.

14. Dispositif selon l'une quelconque des revendications précédentes, dans lequel les impulsions discrètes (x1, ... x4) d'un rayonnement pour le bronzage ont une longueur d'onde d'environ 280 à 300 nanomètres.

15. Dispositif selon l'une quelconque des revendications précédentes, dans lequel les impulsions discrètes (x1, ... x4) d'un rayonnement pour le bronzage ont une longueur d'onde d'environ 290 nanomètres.

16. Dispositif selon la revendication 9 ou selon l'une quelconque des revendications 10 à 15 lorsqu'elles dépendent de la revendications 9, dans lequel les impulsions discrètes d'un rayonnement pour réparer une lésion d'ADN provoquée par un rayonnement ultraviolet dans les cellules de la peau ont une longueur d'onde d'environ 460 à 500 nanomètres.

17. Dispositif selon la revendication 9 ou selon l'une quelconque des revendications 10 à 16 lorsqu'elles dépendent de la revendication 9, dans lequel les impulsions discrètes (x1 ... x4) d'un rayonnement pour la réparation d'une lésion d'ADN provoquée par un rayonnement ultraviolet dans les cellules de la peau ont une longueur d'onde d'environ 480 nanomètres.
